(19) Europäisches Patentamt
European Patent Office
Office européen des brevets

(11) EP 3 483 247 A1

(12) **EUROPEAN PATENT APPLICATION**

(43) Date of publication:
**15.05.2019 Bulletin 2019/20**

(51) Int Cl.:
***C11D 3/38*** *(2006.01)* ***C11D 1/94*** *(2006.01)*
***C11D 11/00*** *(2006.01)* ***C07K 14/36*** *(2006.01)*

(21) Application number: **18178727.6**

(22) Date of filing: **20.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2017 EP 17201320**

(71) Applicant: **The Procter & Gamble Company**
**Cincinnati, OH 45202 (US)**

(72) Inventors:
- **BETTIOL, Jean-Luc Philippe**
  **1853 Strombeek-Bever (BE)**
- **GONZALES, Denis Alfred**
  **1853 Strombeek-Bever (BE)**
- **VELASQUEZ, Juan Esteban**
  **Cincinnati, Ohio 45202 (US)**
- **GEARY, Nicholas William**
  **Cincinnati, Ohio 45202 (US)**

(74) Representative: **Siddiquee, Sanaul Kabir**
**N.V. Procter & Gamble**
**Services Company S.A.**
**Temselaan 100**
**1853 Strombeek-Bever (BE)**

# (54) CLEANING COMPOSITION COMPRISING CHAPLIN PROTEINS

(57) The present invention is directed to a cleaning composition comprising one or more chaplin proteins comprising a DUF320 domain consensus sequence (SEQ ID NO: 1) and have a sequence of less than 350 amino acids and a surfactant system comprising one or more anionic surfactants and one or more co-surfactants selected from the group consisting of amphoteric surfactant, zwitterionic surfactant, and mixtures thereof. Methods of making and using such compositions are also provided.

EP 3 483 247 A1

SEQUENCE LISTING

<110> The Procter & Gamble Company

<120> Cleaning composition

<130> CM04897FM

<160> 23

<170> PatentIn version 3.5

<210> 1
<211> 41
<212> PRT
<213> NONE

<400> 1

```
Ser Pro Gly Val Leu Ser Gly Asn Val Val Gln Val Pro Val Asp Val
1               5                   10                  15


Pro Val Asn Val Cys Gly Asn Thr Val Asn Val Val Gly Leu Leu Asn
            20                  25                  30


Pro Ala Phe Gly Asn Ser Cys Val Asn
        35                  40
```

<210> 2
<211> 252
<212> PRT
<213> Streptomyces coelicolor (strain ATCC BAA-471 / A3(2) / M145)

<400> 2

```
Met Val Ala Ala Ala Ala Ala Thr Gly Ile Leu Ser Leu Cys Gly Ser
1               5                   10                  15


Pro Ala Leu Ala Asp Ser His Ala Asp Gly Ala Ala Thr Asn Ser Pro
            20                  25                  30


Gly Ala Val Ser Gly Asn Ala Leu Gln Val Pro Val Asp Val Pro Val
        35                  40                  45


Asn Ala Cys Gly Asn Thr Val Asp Val Ile Ala Ala Leu Asn Pro Ala
    50                  55                  60


Phe Gly Asn Glu Cys Glu Asn Ala Ser Asp Glu Lys Thr Asp Gly His
65                  70                  75                  80


Gly Gly Gly Tyr Gly Glu Asp Ala Ser Ser Ser Ser Ser Ser Ser Thr
                85                  90                  95


Ser Ala Ser Ser Ser Gly Ser His Ala Asp Gly Ala Thr Glu Gly Ser
```

```
            100                 105                 110

Pro Gly Val Gly Ser Gly Asn Asn Ala Gln Val Pro Val Asp Val Pro
        115                 120                 125

Val Asn Leu Cys Gly Asn Thr Val Asp Val Ile Ala Ala Leu Asn Pro
    130                 135                 140

Val Phe Gly Asn Lys Cys Glu Asn Asp Ala Glu Glu Pro Pro Gly Tyr
145                 150                 155                 160

Gly Glu Glu Glu Pro Pro Pro Pro Thr Thr Pro Pro Gly Tyr Gly Glu
                165                 170                 175

Glu Glu Pro Pro Pro Pro Thr His Glu Glu Pro Pro Pro Pro Ser Gly
            180                 185                 190

Glu Glu Glu Pro Pro Pro Pro Ser Glu Glu Glu His Thr Pro Pro Ala
        195                 200                 205

Pro Gln Thr Glu Gln Pro Pro Ala Leu Ala Glu Thr Gly Ser Glu Gly
    210                 215                 220

Thr Leu Gly Ala Ala Ala Ala Gly Ala Val Leu Ile Ala Gly Gly Ala
225                 230                 235                 240

Ile Leu Tyr Arg Arg Gly Arg Ala Leu Ser Gly Arg
                245                 250


<210>  3
<211>  237
<212>  PRT
<213>  Streptomyces coelicolor (strain ATCC BAA-471 / A3(2) / M145)

<400>  3

Met Arg Arg Val Thr Arg Asn Gly Val Leu Ala Val Ala Ala Ser Gly
1               5                   10                  15

Ala Leu Ala Val Thr Met Pro Ala Tyr Ala Ala Phe Ala Ser Asp Gly
            20                  25                  30

Ala Gly Ala Glu Gly Ser Ala Ala Gly Ser Pro Gly Leu Ile Ser Gly
        35                  40                  45

Asn Thr Val Gln Leu Pro Val Asp Val Pro Val Asp Val Cys Gly Asn
    50                  55                  60

Thr Val Asn Val Val Gly Leu Leu Asn Pro Ala Ala Gly Asn Gly Cys
```

```
65                  70                  75                  80

Ala Asp Ser Gly Glu Pro Gly Ala Ser Tyr Gln Ala Ala Gly Ala Ser
                85                  90                  95

Gly Gly Thr Ser Gly Ser Ala Thr Glu Ala Thr Ser Gly Gly Ala Ala
            100                 105                 110

Ala Glu Gly Ser Gly Lys Asp Ser Pro Gly Val Leu Ser Gly Asn Gly
        115                 120                 125

Val Gln Leu Pro Val His Leu Pro Val Asn Val Ser Gly Asn Ser Val
    130                 135                 140

Asn Val Val Gly Ile Gly Asn Pro Ala Val Gly Asn Glu Ser Thr Asn
145                 150                 155                 160

Asp Ser Gly Asp His Pro Glu Pro Val Arg Pro Pro Ala Glu Pro Glu
                165                 170                 175

Pro Ser Ala Pro Glu Glu Glu Arg Ala Gly Pro Gly Pro Ser Ala His
            180                 185                 190

Ala Ala Pro Pro Arg Glu Glu Val Ser Leu Ala His Thr Gly Thr Asp
        195                 200                 205

Arg Thr Leu Pro Thr Leu Ala Gly Gly Ala Ala Leu Val Leu Gly Gly
    210                 215                 220

Thr Val Leu Tyr Arg Arg Phe Arg Pro Gly Ser Gly Asp
225                 230                 235


<210>  4
<211>  259
<212>  PRT
<213>  Streptomyces coelicolor (strain ATCC BAA-471 / A3(2) / M145)

<400>  4

Met Arg Gln Ala Thr Arg Lys Gly Leu Met Thr Met Ala Ala Ala Thr
1               5                   10                  15

Gly Val Ile Ala Ala Ala Gly Gly Ala Ala His Ala Asp Ser Gly Ala
            20                  25                  30

His Gly Thr Ser Ser Gly Ser Pro Gly Val Leu Ser Gly Asn Thr Val
        35                  40                  45

Gln Ala Pro Val His Val Pro Val Asn Val Cys Gly Asn Thr Val Asp
```

```
    50                  55                  60

Val Val Gly Val Leu Asn Pro Ala Met Gly Asn Ala Cys Ala Asn Gln
65                  70                  75                  80

Gly Gly Gly Ala Ser Gly Gly His Gly Gly His Gly Gly His Gly Gly
                85                  90                  95

Tyr Gly Asp Ser Gly Gly Glu Gly Gly Ser His Gly Gly Ser His Ala
            100                 105                 110

Gly Gly His Ala Thr Asp Ser Pro Gly Val Gly Ser Gly Asn His Val
        115                 120                 125

Glu Val Pro Ile Asp Val Pro Val Asn Val Cys Gly Asn Ser Ile Asp
    130                 135                 140

Val Val Gly Ala Leu Asn Pro Thr Thr Gly Asn Asp Cys Gly Asn Gly
145                 150                 155                 160

Gly Gly Gly Asp His Ser Thr Pro Pro Gly Asp His Glu Thr Pro Pro
                165                 170                 175

Gly Glu Pro His Asn Pro Gly Asn Pro Gly Asn Pro Asp Thr Pro Asp
            180                 185                 190

Lys Pro Ser Gly Pro Asp Asp Glu Thr Pro Gly Asp Ser Thr Asp Gly
        195                 200                 205

Asn Arg Pro Gly Ala Gln Thr Val Asp Gln Pro Arg Gly Asp Ala Ala
    210                 215                 220

Leu Ala Glu Thr Gly Ser Asp Leu Pro Leu Gly Leu Ala Leu Pro Val
225                 230                 235                 240

Gly Ala Gly Ala Leu Leu Ala Gly Thr Val Leu Tyr Arg Lys Ala Arg
                245                 250                 255

Ala Ser Val


<210>  5
<211>  75
<212>  PRT
<213>  Streptomyces coelicolor (strain ATCC BAA-471 / A3(2) / M145)

<400>  5

Met Lys Lys Ser Ala Ala Val Val Ala Gly Ala Ile Met Ala Leu Gly
```

```
1               5                   10                  15

Met Ala Ala Pro Ala Phe Ala Asp Ala Gly Ala Glu Gly Ala Ala Val
            20                  25                  30

Gly Ser Pro Gly Val Leu Ser Gly Asn Val Ile Gln Val Pro Val His
        35                  40                  45

Val Pro Val Asn Val Cys Gly Asn Ser Ile Asn Val Val Gly Leu Leu
    50                  55                  60

Asn Pro Ala Phe Gly Asn Lys Cys Glu Asn Asp
65                  70                  75


<210>  6
<211>  82
<212>  PRT
<213>  Streptomyces coelicolor (strain ATCC BAA-471 / A3(2) / M145)

<400>  6

Met Lys Asn Leu Lys Lys Ala Ala Ala Val Thr Met Val Ala Gly Gly
1               5                   10                  15

Leu Ile Ala Ala Gly Ala Gly Met Ala Ser Ala Thr Asp Gly Gly Ala
            20                  25                  30

His Ala His Gly Lys Ala Val Gly Ser Pro Gly Val Ala Ser Gly Asn
        35                  40                  45

Leu Val Gln Ala Pro Ile His Ile Pro Val Asn Ala Val Gly Asn Ser
    50                  55                  60

Val Asn Val Ile Gly Val Leu Asn Pro Ala Phe Gly Asn Leu Gly Val
65                  70                  75                  80

Asn His


<210>  7
<211>  88
<212>  PRT
<213>  Streptomyces coelicolor (strain ATCC BAA-471 / A3(2) / M145)

<400>  7

Met Tyr Asn Pro Lys Glu His Phe Ser Met Ser Arg Ile Ala Lys Gly
1               5                   10                  15

Leu Ala Leu Thr Ser Val Ala Ala Ala Ala Val Ala Gly Thr Ala Gly
            20                  25                  30
```

```
Val Ala Ala Ala Asp Ser Gly Ala Gln Ala Ala Ala Ala His Ser Pro
        35                  40                  45

Gly Val Leu Ser Gly Asn Val Val Gln Val Pro Val His Ile Pro Val
    50                  55                  60

Asn Val Cys Gly Asn Thr Ile Asp Val Ile Gly Leu Leu Asn Pro Ala
65                  70                  75                  80

Phe Gly Asn Glu Cys Glu Asn Asp
                85


<210>  8
<211>  90
<212>  PRT
<213>  Streptomyces coelicolor (strain ATCC BAA-471 / A3(2) / M145)

<400>  8

Met Ser Arg Ile Ala Lys Ala Ala Gly Val Ala Leu Gly Thr Gly Ala
1               5                   10                  15

Val Val Leu Ser Gly Thr Gly Met Ala Met Ala Asp Ala Gly Ala Ala
            20                  25                  30

Gly Ala Ala Val Gly Ser Pro Gly Val Leu Ser Gly Asn Val Val Gln
        35                  40                  45

Val Pro Val His Val Pro Val Asn Leu Cys Gly Asn Thr Ile Asp Val
    50                  55                  60

Ile Gly Leu Leu Asn Pro Ala Phe Gly Asn Ala Cys Glu Asn Gly Asp
65                  70                  75                  80

Asp Asp Asp Lys Ser Gly Gly Tyr Gly Gly
                85                  90


<210>  9
<211>  77
<212>  PRT
<213>  Streptomyces coelicolor (strain ATCC BAA-471 / A3(2) / M145)

<400>  9

Met Leu Lys Lys Val Val Ala Ala Ala Ala Ala Thr Gly Gly Leu Val
1               5                   10                  15

Leu Ala Gly Ala Gly Met Ala Val Ala Asp Ser Gly Ala Gln Gly Ala
            20                  25                  30
```

Ala Val His Ser Pro Gly Val Leu Ser Gly Asn Val Val Gln Val Pro
35 40 45

Val His Val Pro Val Asn Val Cys Gly Asn Thr Ile Ser Val Ile Gly
50 55 60

Leu Leu Asn Pro Ala Phe Gly Asn Val Cys Ile Asn Lys
65 70 75

<210> 10
<211> 79
<212> PRT
<213> Catenulispora acidiphila (strain DSM 44928 / NRRL B-24433 / NBRC 102108 / JCM 14897)

<400> 10

Met Leu Lys Thr Lys Lys Ile Ala Ala Leu Val Ala Ala Thr Gly Gly
1 5 10 15

Leu Val Met Ala Gly Ala Gly Met Ala Ser Ala Glu Ala Thr Ala Gly
20 25 30

Gly Ser Ser Val Gly Ser Pro Gly Ile Val Ser Gly Asn Thr Ile Gln
35 40 45

Val Pro Val His Val Pro Val Asn Ala Cys Gly Leu Thr Val Ser Val
50 55 60

Ile Gly Ile Leu Asp Gln Ala Phe Gly Asn Thr Cys Val Asn Gly
65 70 75

<210> 11
<211> 79
<212> PRT
<213> Catenulispora acidiphila (strain DSM 44928 / NRRL B-24433 / NBRC 102108 / JCM 14897)

<400> 11

Met Ser Met Arg Lys Thr Leu Val Ala Ala Ala Phe Ala Ala Val Ala
1 5 10 15

Val Leu Gly Thr Ala Gly Thr Ala Ser Ala Ser Gly Ala Gly Ala Ile
20 25 30

Gly Gly Ala Ile Gly Ser Pro Gly Leu Leu Ser Gly Asn Asn Ile Gln
35 40 45

Ile Pro Ile Asn Ile Pro Ile Asn Leu Cys Gly Asn Asp Ile Ser Val
50 55 60

```
Leu Ala Ala Leu Thr Gly Ala Ala Gly Asn Thr Cys Val Asn Tyr
65                  70                  75


<210>  12
<211>  75
<212>  PRT
<213>  Streptomyces coelicolor (strain ATCC BAA-471 / A3(2) / M145)

<400>  12

Met Lys Lys Ser Ala Ala Val Val Ala Gly Ala Ile Met Ala Leu Gly
1               5                   10                  15


Met Ala Ala Pro Ala Phe Ala Asp Ala Gly Ala Glu Gly Ala Ala Val
            20                  25                  30


Gly Ser Pro Gly Val Leu Ser Gly Asn Val Ile Gln Val Pro Val His
        35                  40                  45


Val Pro Val Asn Val Cys Gly Asn Ser Ile Asn Val Val Gly Leu Leu
    50                  55                  60


Asn Pro Ala Phe Gly Asn Lys Cys Glu Asn Asp
65                  70                  75


<210>  13
<211>  89
<212>  PRT
<213>  Streptomyces pristinaespiralis ATCC 25486

<400>  13

Met Ser Arg Ile Ala Lys Ala Ala Ala Val Val Ala Gly Thr Gly Ala
1               5                   10                  15


Ile Leu Ala Gly Gly Ala Gly Met Ala Ala Ala Asp Ala Gly Ala His
            20                  25                  30


Gly Gly Ala Ala Gly Ser Pro Gly Val Val Ser Gly Asn Ala Val Gln
        35                  40                  45


Val Pro Val His Val Pro Val Asn Val Cys Gly Asn Thr Val Asn Val
    50                  55                  60


Ile Ala Leu Leu Asn Pro Thr Phe Gly Asn Gln Cys Ala Asn Val Asp
65                  70                  75                  80


Gly Gly Asp His Gly Ala Tyr Gly Gly
                85
```

<210> 14
<211> 340
<212> PRT
<213> Catenulispora acidiphila (strain DSM 44928 / NRRL B-24433 / NBRC 102108 / JCM 14897)

<400> 14

```
Met Gln Ser Gln Val Lys Arg Arg Ile Val Phe Gly Leu Thr Thr Gly
1               5                   10                  15

Gly Met Leu Ala Thr Gly Gly Val Gly Leu Ala His Ala Asp Ala Ala
            20                  25                  30

Ala Ala Gly Val Gly Asp Gly Ala Thr Thr Ala Gly Ser Pro Gly Ile
        35                  40                  45

Leu Ser Gly Asn Thr Ile Gln Ile Pro Val Asn Ile Pro Ile Asn Val
    50                  55                  60

Cys Gly Val Thr Ala Asn Val Val Gly Leu Leu Asn Pro Ala Glu Gly
65                  70                  75                  80

Asn His Cys Ala Asn Ser Gly Gly Ala Thr Ala Asn Gly Gly Gly Pro
                85                  90                  95

Ser Ser Gly Gly Ala Ser Ala Ser Gly Ser Ser Val Gly Ser Pro Gly
            100                 105                 110

Ile Leu Ser Gly Asn Thr Ile Gln Ala Pro Val Arg Val Pro Val Asn
        115                 120                 125

Ala Cys Gly Asp Thr Val Asn Val Val Gly Val Gly Asn Gly Ala Lys
    130                 135                 140

Gly Asn His Cys Ala Asn Glu Gly Gly Thr Thr Gly Gly Gly Ala Thr
145                 150                 155                 160

Ala Thr Gly Ser Ser Val Gly Ser Pro Gly Ile Ile Ser Gly Asn Thr
                165                 170                 175

Val Gln Val Pro Val Asn Val Pro Val Asn Leu Cys Gly Asp Thr Val
            180                 185                 190

Asn Val Val Gly Val Gly Asn Asn Ala Asp Gly Asn His Cys Leu Asn
        195                 200                 205

Ala Gly Gly Gly Ala Val Thr Gly Gly Ser Thr Ala Thr Gly Ser Ser
    210                 215                 220
```

Val Gly Ser Pro Gly Ile Val Ser Gly Asn Thr Ile Gln Leu Pro Ile
225                 230                 235                 240

Ser Ile Pro Val Asn Val Cys Gly Asp Ser Val Asn Val Val Gly Ile
                245                 250                 255

Ala Asn Gly Ala Ala Gly Asn Ala Cys Ala Asn Asp Thr Pro Ala Pro
            260                 265                 270

Pro Thr Val Thr Pro Pro Pro Thr Thr Thr Thr Gly Trp Thr Ala Pro
        275                 280                 285

Arg Thr Ala Pro Thr Glu Thr Gly Thr Ala Val Pro Ala Ala Thr Gly
    290                 295                 300

Met Leu Ala His Thr Gly Ala Asp Gly Leu Met Leu Ala Pro Leu Gly
305                 310                 315                 320

Ala Ala Leu Met Gly Gly Gly Ala Phe Met Tyr Arg Lys Tyr Lys Pro
                325                 330                 335

Arg Arg Met Phe
            340

<210>  15
<211>  267
<212>  PRT
<213>  Catenulispora acidiphila (strain DSM 44928 / NRRL B-24433 / NBRC 102108 / JCM 14897)

<400>  15

Met Leu Asn Pro Ala Glu Gly Asn His Cys Ala Asn Ser Gly Gly Ala
1               5                   10                  15

Thr Ala Asn Gly Gly Gly Pro Ser Ser Gly Gly Ala Ser Ala Ser Gly
            20                  25                  30

Ser Ser Val Gly Ser Pro Gly Ile Leu Ser Gly Asn Thr Ile Gln Ala
        35                  40                  45

Pro Val Arg Val Pro Val Asn Ala Cys Gly Asp Thr Val Asn Val Val
    50                  55                  60

Gly Val Gly Asn Gly Ala Lys Gly Asn His Cys Ala Asn Glu Gly Gly
65                  70                  75                  80

Thr Thr Gly Gly Gly Ala Thr Ala Thr Gly Ser Ser Val Gly Ser Pro

85 90 95

Gly Ile Ile Ser Gly Asn Thr Val Gln Val Pro Val Asn Val Pro Val
100 105 110

Asn Leu Cys Gly Asp Thr Val Asn Val Val Gly Val Gly Asn Asn Ala
115 120 125

Asp Gly Asn His Cys Leu Asn Ala Gly Gly Gly Ala Val Thr Gly Gly
130 135 140

Ser Thr Ala Thr Gly Ser Ser Val Gly Ser Pro Gly Ile Val Ser Gly
145 150 155 160

Asn Thr Ile Gln Leu Pro Ile Ser Ile Pro Val Asn Val Cys Gly Asp
165 170 175

Ser Val Asn Val Val Gly Ile Ala Asn Gly Ala Ala Gly Asn Ala Cys
180 185 190

Ala Asn Asp Thr Pro Ala Pro Pro Thr Val Thr Pro Pro Pro Thr Thr
195 200 205

Thr Thr Gly Trp Thr Ala Pro Arg Thr Ala Pro Thr Glu Thr Gly Thr
210 215 220

Ala Val Pro Ala Ala Thr Gly Met Leu Ala His Thr Gly Ala Asp Gly
225 230 235 240

Leu Met Leu Ala Pro Leu Gly Ala Ala Leu Met Gly Gly Gly Ala Phe
245 250 255

Met Tyr Arg Lys Tyr Lys Pro Arg Arg Met Phe
260 265

<210> 16
<211> 52
<212> PRT
<213> Streptomyces coelicolor (strain ATCC BAA-471 / A3(2) / M145)

<400> 16

Asp Ala Gly Ala Glu Gly Ala Ala Val Gly Ser Pro Gly Val Leu Ser
1 5 10 15

Gly Asn Val Ile Gln Val Pro Val His Val Pro Val Asn Val Cys Gly
20 25 30

Asn Ser Ile Asn Val Val Gly Leu Leu Asn Pro Ala Phe Gly Asn Lys

```
        35                  40                  45


Cys Glu Asn Asp
    50


<210>  17
<211>  55
<212>  PRT
<213>  Streptomyces coelicolor (strain ATCC BAA-471 / A3(2) / M145)

<400>  17

Thr Asp Gly Gly Ala His Ala His Gly Lys Ala Val Gly Ser Pro Gly
1               5                   10                  15


Val Ala Ser Gly Asn Leu Val Gln Ala Pro Ile His Ile Pro Val Asn
            20                  25                  30


Ala Val Gly Asn Ser Val Asn Val Ile Gly Val Leu Asn Pro Ala Phe
        35                  40                  45


Gly Asn Leu Gly Val Asn His
    50                  55


<210>  18
<211>  52
<212>  PRT
<213>  Streptomyces coelicolor (strain ATCC BAA-471 / A3(2) / M145)

<400>  18

Asp Ser Gly Ala Gln Ala Ala Ala Ala His Ser Pro Gly Val Leu Ser
1               5                   10                  15


Gly Asn Val Val Gln Val Pro Val His Ile Pro Val Asn Val Cys Gly
            20                  25                  30


Asn Thr Ile Asp Val Ile Gly Leu Leu Asn Pro Ala Phe Gly Asn Glu
        35                  40                  45


Cys Glu Asn Asp
    50


<210>  19
<211>  63
<212>  PRT
<213>  Streptomyces coelicolor (strain ATCC BAA-471 / A3(2) / M145)

<400>  19

Asp Ala Gly Ala Ala Gly Ala Ala Val Gly Ser Pro Gly Val Leu Ser
1               5                   10                  15
```

Gly Asn Val Val Gln Val Pro Val His Val Pro Val Asn Leu Cys Gly
20 25 30

Asn Thr Ile Asp Val Ile Gly Leu Leu Asn Pro Ala Phe Gly Asn Ala
35 40 45

Cys Glu Asn Gly Asp Asp Asp Asp Lys Ser Gly Gly Tyr Gly Gly
50 55 60

<210> 20
<211> 52
<212> PRT
<213> Streptomyces coelicolor (strain ATCC BAA-471 / A3(2) / M145)

<400> 20

Asp Ser Gly Ala Gln Gly Ala Ala Val His Ser Pro Gly Val Leu Ser
1 5 10 15

Gly Asn Val Val Gln Val Pro Val His Val Pro Val Asn Val Cys Gly
20 25 30

Asn Thr Ile Ser Val Ile Gly Leu Leu Asn Pro Ala Phe Gly Asn Val
35 40 45

Cys Ile Asn Lys
50

<210> 21
<211> 104
<212> PRT
<213> Conidiobolus coronatus (strain ATCC 28846 / CBS 209.66 / NRRL 28638) (Delacroixia coronata)

<400> 21

Met Gln Leu Leu Ile Leu Ala Ser Leu Ile Ala Phe Ile Ala Ser Ala
1 5 10 15

Pro Val Ala Asn Gly Gly Asp Phe Lys Ser His Gly His Ile Ala Gly
20 25 30

Asn Asn Ile Arg Gly Ser Val His Ala Pro Gln Asn Thr Cys Asn Asn
35 40 45

Gly Val Lys Val Ile Gly Gly Thr Asn Thr Ser Phe Asp Asn Ser Cys
50 55 60

Thr Asn Thr Ser Val Glu Ser Asp Thr Asp Asp His Ser Asn Ser Asp
65 70 75 80

```
Thr Asp Asp His Ser Asn Ser Asp Thr Asp Asp His Ser Asn Ser Glu
                85                  90                  95

Asp Glu Ser Gly Ser Asp Asp Ser
            100
```

<210> 22
<211> 156
<212> DNA
<213> None

<400> 22

```
gatagtggtg ctcaagcggc ggctgcccat agtccgggtg tcctgtctgg taatgtggtt       60

caagttccgg ttcatattcc ggttaatgtg tgcggcaata cgattgatgt gattggtctg      120

ctgaacccgg ccttcggtaa tgaatgtgaa aatgat                                156
```

<210> 23
<211> 456
<212> PRT
<213> Streptomyces coelicolor A3(2)

<400> 23

```
Met Gly Ser Ser His His His His His His Gly Thr Lys Thr Glu Glu
1               5                   10                  15

Gly Lys Leu Val Ile Trp Ile Asn Gly Asp Lys Gly Tyr Asn Gly Leu
            20                  25                  30

Ala Glu Val Gly Lys Lys Phe Glu Lys Asp Thr Gly Ile Lys Val Thr
        35                  40                  45

Val Glu His Pro Asp Lys Leu Glu Glu Lys Phe Pro Gln Val Ala Ala
    50                  55                  60

Thr Gly Asp Gly Pro Asp Ile Ile Phe Trp Ala His Asp Arg Phe Gly
65                  70                  75                  80

Gly Tyr Ala Gln Ser Gly Leu Leu Ala Glu Ile Thr Pro Asp Lys Ala
                85                  90                  95

Phe Gln Asp Lys Leu Tyr Pro Phe Thr Trp Asp Ala Val Arg Tyr Asn
            100                 105                 110

Gly Lys Leu Ile Ala Tyr Pro Ile Ala Val Glu Ala Leu Ser Leu Ile
        115                 120                 125

Tyr Asn Lys Asp Leu Leu Pro Asn Pro Pro Lys Thr Trp Glu Glu Ile
    130                 135                 140
```

```
Pro Ala Leu Asp Lys Glu Leu Lys Ala Lys Gly Lys Ser Ala Leu Met
145                 150                 155                 160

Phe Asn Leu Gln Glu Pro Tyr Phe Thr Trp Pro Leu Ile Ala Ala Asp
                165                 170                 175

Gly Gly Tyr Ala Phe Lys Tyr Glu Asn Gly Lys Tyr Asp Ile Lys Asp
            180                 185                 190

Val Gly Val Asp Asn Ala Gly Ala Lys Ala Gly Leu Thr Phe Leu Val
        195                 200                 205

Asp Leu Ile Lys Asn Lys His Met Asn Ala Asp Thr Asp Tyr Ser Ile
    210                 215                 220

Ala Glu Ala Ala Phe Asn Lys Gly Glu Thr Ala Met Thr Ile Asn Gly
225                 230                 235                 240

Pro Trp Ala Trp Ser Asn Ile Asp Thr Ser Lys Val Asn Tyr Gly Val
                245                 250                 255

Thr Val Leu Pro Thr Phe Lys Gly Gln Pro Ser Lys Pro Phe Val Gly
            260                 265                 270

Val Leu Ser Ala Gly Ile Asn Ala Ala Ser Pro Asn Lys Glu Leu Ala
        275                 280                 285

Lys Glu Phe Leu Glu Asn Tyr Leu Leu Thr Asp Glu Gly Leu Glu Ala
    290                 295                 300

Val Asn Lys Asp Lys Pro Leu Gly Ala Val Ala Leu Lys Ser Tyr Glu
305                 310                 315                 320

Glu Glu Leu Ala Lys Asp Pro Arg Ile Ala Ala Thr Met Glu Asn Ala
                325                 330                 335

Gln Lys Gly Glu Ile Met Pro Asn Ile Pro Gln Met Ser Ala Phe Trp
            340                 345                 350

Tyr Ala Val Arg Thr Ala Val Ile Asn Ala Ala Ser Gly Arg Gln Thr
        355                 360                 365

Val Asp Glu Ala Leu Lys Asp Ala Gln Thr Gly Thr Asp Tyr Asp Ile
    370                 375                 380

Pro Thr Thr Lys Leu Gly Ser Gly Ser Ser Gly Ser Gly Glu Asn Leu
```

```
385                 390                 395                 400

Tyr Phe Gln Gly Asp Ser Gly Ala Gln Ala Ala Ala Ala His Ser Pro
                405                 410                 415

Gly Val Leu Ser Gly Asn Val Val Gln Val Pro Val His Ile Pro Val
            420                 425                 430

Asn Val Cys Gly Asn Thr Ile Asp Val Ile Gly Leu Leu Asn Pro Ala
        435                 440                 445

Phe Gly Asn Glu Cys Glu Asn Asp
    450                 455
```

## Description

REFERENCE TO A SEQUENCE LISTING

This application contains Sequence Listings in computer readable form. The computer readable form is incorporated herein by reference.

FIELD OF THE INVENTION

The present invention relates to a cleaning composition comprising a specific surfactant system and one or more chaplin proteins. The composition provides one or more benefits, including good cleaning particularly good grease emulsification, long lasting suds especially in presence of greasy soils and surface modification that can contribute to second time cleaning benefits, improved drying, improved shine in the case of dishware.

BACKGROUND OF THE INVENTION

Cleaning compositions should have a good suds profile in particular a long lasting suds profile while providing good soil and grease cleaning. Users usually see suds as an indicator of the performance of the cleaning composition. Moreover, the user of a cleaning composition may also use the suds profile and the appearance of the suds (e.g. density, whiteness) as an indicator that the wash solution still contains active cleaning ingredients. This is particularly the case for manual washing, also referred to herein as hand-washing, where the user usually doses the cleaning composition depending on the suds remaining and renews the wash solution when the suds subside or when the suds does not look thick enough. Thus, a cleaning composition, particularly a manual wash cleaning composition that generates little or low density suds would tend to be replaced by the user more frequently than is necessary. Accordingly, it is desirable for a cleaning composition to provide "good sudsing profile", which includes good suds height and/or density as well as good suds duration during the initial mixing of the composition with water and during the entire washing operation.

Several families of natural surface active proteins are able to produce suds in aqueous solutions (*see* Cooper, A., et al. (2017), Colloids Surf., A: Physiochemical and Engineering Aspects; Schor, M., et al. (2016), Trends Biochem. Sci. 41(7): 610-620). However, the amount of sudsing generated by such surface active proteins in cleaning formulations is limited.

Accordingly, the need remains for an improved cleaning composition comprising surface active proteins which has a further improved sudsing profile, particularly at low proteins concentrations in the cleaning compositions. The need also exists for an improved cleaning composition, when used in a manual-washing process, the composition preferably also provides a pleasant washing experience, *i.e*, good feel on the user's hands during the wash. Preferably the cleaning compositions are also easy to rinse. Preferably in addition, the composition provides a good finish to the washed items. There is also the desire to reduce the amount of surfactants without negatively impacting sudsing nor grease cleaning and emulsification profile. Thus, there is the need to find new compositions that improve suds longevity in hand washing conditions. The Applicant discovered that some or all of the above-mentioned needs can be at least partially fulfilled through the improved cleaning composition as described herein below.

SUMMARY OF THE INVENTION

The present invention meets one or more of these needs based on the surprising discovery that by formulating a cleaning composition comprising a specific surfactant system and one or more chaplin proteins, such a composition exhibits good sudsing profile, particularly desirable suds volume and/or sustained suds stabilization, especially in the presence of greasy soils. It also provides good grease cleaning and emulsification benefits and can also provide surface modifications facilitating next time cleaning benefit.

According to a first aspect, the present invention is directed to a cleaning composition comprising from 1 wt% to 60 wt%, preferably from 5 wt% to 50 wt% by weight of said composition of a specific surfactant system and from 0.001 wt% to 5 wt%, preferably from 0.1 wt% to 1 wt%, by weight of said composition, based on active protein, of one or more chaplin proteins, wherein the chaplin proteins comprise a DUF320 domain consensus sequence (SEQ ID NO: 1) and have a sequence of less than 350 amino acids. The surfactant system comprises one or more anionic surfactants and one or more co-surfactants selected from the group consisting of amphoteric surfactant, zwitterionic surfactant, and mixtures thereof.

Preferably the cleaning composition is a manual-washing cleaning composition. Preferably the cleaning composition is for manual dishwashing. Preferred compositions are in the form of a liquid.

In another aspect, the present invention is directed to a method comprising contacting a cleaning composition of the invention with a surface, preferably a hard surface, preferably dishware. The composition provides good grease

removal from all types of surfaces, preferably from hard surfaces, preferably dishware.

In another aspect, the present invention is directed to a method of manually washing dishware comprising the steps of delivering a detergent composition of the invention into a volume of water to form a wash solution and immersing the dishware in the solution.

In yet another aspect, the present invention is directed to a method of manually washing soiled items, preferably hard surfaces more preferably dishware, comprising contacting a cleaning composition of the invention, wherein said composition modifies the hydrophobicity of said surface, preferably hard surface more preferably dishware, as a result of said contacting step.

In yet another aspect, the present invention is directed to a method of improving suds longevity and/or grease emulsification in a washing process for washing soiled articles, preferably dishware. The method comprises the steps of: a) delivering a cleaning composition of the invention to a volume of water to form a wash liquor; and b) immersing the soiled articles into said wash liquor.

In yet another aspect, the present invention relates to a method of manually washing dishware comprising: i) delivering a composition as described herein above onto the dishware or a cleaning implement; ii) cleaning the dishware with the composition in the presence of water; and iii) optionally, rinsing the dishware. Preferably, the composition of the present invention is used in neat form (*i.e.,* direct application) since greater benefits in terms of grease cleaning are obtained when the composition is directly applied on the soiled surface or on a cleaning implement, such as a sponge, to be used to clean the soiled surface.

In yet another aspect, the present invention is directed to use of one or more chaplin proteins of the invention to provide improved suds longevity and/or improved grease emulsification in an aqueous wash liquor during a washing process.

It is an object of the composition of the present invention to exhibit good sudsing profile, preferably high suds volume and sustained suds aesthetics (*i.e.*, whiteness, consistency).

It is an object of the composition of the present invention to exhibit good sudsing profile, preferably stable suds during a substantial portion of or for the entire manual washing preferably dishwashing process.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## DETAILED DESCRIPTION OF THE INVENTION

### Definitions

As used herein, the articles "a" and "an" when used in a claim, are understood to mean one or more of what is claimed or described.

As used herein, the term "amino acid identity" means the identity between two or more amino acid sequences and is expressed in terms of the identity or similarity between the sequences. Sequence identity can be measured in terms of percentage identity; the higher the percentage, the more identical the sequences are. The percentage identity is calculated over the length of comparison. For example, the amino acid identity is typically calculated over the entire length of a sequence aligned against the entire length of the reference sequence (e.g., SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 17, SEQ ID NO: 18, SEQ ID NO: 19, SEQ ID NO: 20, SEQ ID NO: 21 or SEQ ID NO: 23). Methods of alignment of sequences for comparison are well known in the art and identity can be calculated by many known methods. Various programs and alignment algorithms are described in the art. It should be noted that the terms 'sequence identity' and 'sequence similarity' can be used interchangeably.

As used herein, the term "cleaning composition" refers to a composition or formulation designed for cleaning soiled surfaces. Such compositions include but are not limited to, dishwashing compositions, laundry detergent compositions, fabric softening compositions, fabric enhancing compositions, fabric freshening compositions, laundry pre-wash, laundry pretreat, laundry additives, spray products, dry cleaning agent or composition, laundry rinse additive, wash additive, post-rinse fabric treatment, ironing aid, hard surface cleaning compositions, unit dose formulation, delayed delivery formulation, detergent contained on or in a porous substrate or nonwoven sheet, and other suitable forms that may be apparent to one skilled in the art in view of the teachings herein. Such compositions may be used as a pre-cleaning treatment, a post-cleaning treatment, or may be added during the rinse or wash cycle of the cleaning process. The cleaning compositions may have a form selected from liquid, powder, single-phase or multi-phase unit dose or pouch form, tablet, gel, paste, bar, or flake. Preferably the composition is for manual-washing. Preferably, the cleaning composition of the present invention is a dishwashing detergent. Preferably the composition is in the form of a liquid.

As used herein the term "fragment" means an amino acid sequence of at least 30, 60, 100, 150 contiguous amino acids of the reference sequences or any integer there between.

As used herein the term "improved suds longevity" means an increase in the duration of visible suds in a washing process cleaning soiled articles using the composition comprising one or more chaplin proteins, compared with the suds longevity provided by the same composition and process in the absence of the chaplin proteins.

As used herein, the term "next time cleaning benefit" means the surface to be cleaned could be treated with a composition which would assist in easier removal of soil and/or stains during subsequent cleaning.

As used herein, the term "soiled surfaces" refers non-specifically to any type of flexible material consisting of a network of natural or artificial fibers, including natural, artificial, and synthetic fibers, such as, but not limited to, cotton, linen, wool, polyester, nylon, silk, acrylic, and the like, as well as various blends and combinations. Soiled surfaces may further refer to any type of hard surface, including natural, artificial, or synthetic surfaces, such as, but not limited to, tile, granite, grout, glass, composite, vinyl, hardwood, metal, cooking surfaces, plastic, and the like, as well as blends and combinations, as well as dishware. Key targeted soiled surfaces by this application are soiled dishware.

As used herein, the term "variant" of the chaplin proteins means an amino acid sequence when the chaplin protein is modified by, or at, one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type chaplin protein, such as for example, a protein with a truncated N-terminus. Variants may also include forms derived by adding an extra amino acid sequence to a wild-type protein, such as for example, an N-terminal tag, a C-terminal tag or an insertion in the middle of the protein sequence.

As used herein, the term "water hardness" or "hardness" means uncomplexed cation ions (*i.e.*, $Ca^{2+}$ or $Mg^{2+}$) present in water that have the potential to precipitate with anionic surfactants or other anionic actives in the cleaning composition under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

## Cleaning Composition

The present invention envisages a cleaning composition, preferably a hand dishwashing cleaning composition, comprising a specific surfactant system and one or more chaplin proteins. The composition of the invention provides very good suds duration especially in presence of fatty and/or oily soils. The invention also envisages a method of hand dishwashing and use of the composition for prolonging suds duration.

A preferred cleaning composition is a manual dishwashing composition, preferably in liquid form. It typically contains from 30% to 95%, preferably from 40% to 90%, more preferably from 50% to 85% by weight of the composition of a liquid carrier in which the other essential and optional components are dissolved, dispersed or suspended. One preferred component of the liquid carrier is water.

Preferably the pH of the cleaning composition of the invention, measured as a 10% product concentration in demineralized water at 20°C, is adjusted to between 3 and 14, more preferably between 4 and 13, more preferably between 6 and 12 and most preferably between 8 and 10. The pH of the cleaning composition can be adjusted using pH modifying ingredients known in the art.

## Chaplin Proteins

The cleaning composition in accordance with the present invention comprises one or more chaplin proteins. Chaplin proteins (coelicolor hydrophobic aerial proteins) were originally discovered in *Streptomyces coelicolor,* but genome mining in protein databases indicate that these proteins are broadly distributed, including species in *Actinobacteria*, *Cyanobacteria*, *Firmicutes*, and even Fungi.

Chaplin proteins share significant sequence identity, including a highly conserved chaplin domain of approximately 40 amino acids, usually referred as DUF320 (pfam03777). The consensus sequence of the DUF320 domain is shown in SEQ ID NO: 1. A "chaplin protein" of the present invention is any protein containing at least one DUF320 domain and with a length of less than about 350 amino acids

Proteins containing multiple DUF320 domains have been deposited on protein sequence databases. *S. coelicolor* A32 produces eight different chaplins (ChpA-H). For example, *S. coelicolor* ChpA (SEQ ID NO: 2), *S. coelicolor*

ChpB (SEQ ID NO: 3) and *S. coelicolor* ChpC (SEQ ID NO: 4) contain two N-terminal DUF320 domains and a C-terminal cell wall anchoring domain, whereas *S. coelicolor* ChpD (SEQ ID NO: 5), *S. coelicolor* ChpE (SEQ ID NO: 6), *S. coelicolor* ChpF (SEQ ID NO: 7), *S. coelicolor* ChpG (SEQ ID NO: 8) and *S. coelicolor* ChpH (SEQ ID NO: 9) are shorter and contain an N-terminal secretion signal peptide and a C-terminal DUF320 domain. Other species of *Streptomyces* also produce chaplins. For example, a predicted chaplin from *S. pristinaespiralis* (SEQ ID NO: 13) contain an N-terminal signal peptide, a DUF320 domain, and an extra few amino acids at the C-terminus with unknown function.

Other bacterial species, e.g. *Catenulispora acidiphila*, are predicted to produce several chaplins with different domain architectures. Similarly to ChpD-H from *S. coelicolor,* two predicted *C. acidiphila* chaplins (SEQ ID NO: 10 and SEQ ID NO: 11) are short and contain only an N-terminal secretion signal peptide and a C-terminal DUF320 domain. Another *C. acidiphila* chaplin (SEQ ID NO: 14) contains an N-terminal secretion signal peptide, four DUF320 domains, and a C-terminal cell wall anchoring domain. Chaplins with different domain architecture are part of the current invention.

Even though several amino acids are highly conserved in different chaplins, the sequence identity of chaplins can be pretty low. For example, the predicted chaplin from *Conidiobolus coronatus* (SEQ ID NO: 21) has between 18% and 21% sequence identity when compared to ChpD-H.

Frequently, chaplin proteins contain two cysteine residues (e.g., ChpD, ChpF, ChpG, and ChpH) that may be involved in disulfide bond formation, perhaps enabling heteropolymerization and creating longer structures. In other examples, the cysteine residues are not present (e.g., ChpE). Chaplins with or without cysteine residues are part of the current invention. Furthermore, a diverse number of proteins contain the DUF320 domain in combination with other domains, which may add different functions. These proteins are also part of the current invention.

The role of chaplin proteins in *S. coelicolor* is to coat the aerial hyphae assisting spore dispersal and colonization of surrounding soil, while different chaplin proteins can adopt distinct roles *in vivo.* For example, *S. coelicolor* ChpE and ChpH are expressed at high levels in the vegetative and aerial mycelial phases and likely perform two different functions: lowering the surface tension of water (*i.e.,* as surfactants) and assembling into a hydrophobic layer to coat the emerging hyphae. In contrast, the other chaplin proteins are only expressed during the aerial hyphae formation and may only contribute to the later role.

Unexpectedly, the Applicants found that chaplin proteins are able to increase sudsing in the presence of a specific surfactant system. Not wishing to be bound by theory, the Applicants believe that the increased sudsing benefits are due to the specific amino acid sequences and/or protein structures enhancing the adsorption at the interface between two phases (oil/water or air/water).

Accordingly, a cleaning composition of the present invention comprises: a) from 1 wt% to 60 wt%, preferably from 5 wt% to 50 wt%, by weight of the cleaning composition of a surfactant system comprising one or more anionic surfactants and one or more co-surfactants selected from the group consisting of amphoteric surfactant, zwitterionic surfactant, and mixtures thereof; and b) from 0.001 wt% to 5 wt%, preferably from 0.1 wt% to 1 wt%, by weight of the cleaning composition, based on active protein, of one or more chaplin proteins, wherein the chaplin proteins comprise a DUF320 domain consensus sequence (SEQ ID NO: 1) and have a sequence of less than 350 amino acids.

Preferably the chaplin proteins have at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23, more preferably to at least one wild-type protein sequence selected from the group consisting of: *Streptomyces coelicolor* ChpD (SEQ ID NO: 16), *Streptomyces coelicolor* ChpE (SEQ ID NO: 17), *Streptomyces coelicolor* ChpF (SEQ ID NO: 18), *Streptomyces coelicolor* ChpG (SEQ ID NO: 19), and *Streptomyces coelicolor* ChpH (SEQ ID NO: 20), more preferably *Streptomyces coelicolor* ChpE (SEQ ID NO: 17), and *Streptomyces coelicolor* ChpF (SEQ ID NO: 18).

Preferably the chaplin proteins have at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one reference sequence, preferably one wild-type protein sequence, selected from the group consisting of: SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, and SEQ ID NO: 15.

Preferably, the chaplin proteins have at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of the DUF320 consensus sequence SEQ ID NO: 1.

The invention also includes chaplin protein variants. For example, chaplin protein variants, as used herein, include a sequence resulting when a wild-type protein is modified by, or at, one or more amino acids (for example 1, 2,

5 or 10 amino acids). The invention also includes chaplin protein variants in the form of truncated forms derived from a wild-type chaplin, such as a wild-type chaplin protein with a truncated N-terminus or a truncated C-terminus.

Majority of chaplin proteins are predicted to include an N-terminal signal peptide that is likely removed upon secretion by the native organisms. Preferably the chaplin protein variants of the present invention are without the N-terminal signal peptide. For example, SEQ ID NO: 16, SEQ ID NO: 17, SEQ ID NO: 18, SEQ ID NO: 19, and SEQ ID NO: 20 are variants of the full length wild-type *Streptomyces coelicolor* ChpD-H (SEQ ID NO: 5, SEQ ID NO: 6, SEQ ID NO: 7, SEQ ID NO: 8, and SEQ ID NO: 9, respectively) without the N-terminal signal peptide. Bioinformatic tools, such as SignalP version 4.1 (Petersen TN., Brunak S., von Heijne G. and Nielsen H. (2011), Nature Methods, 8:785-786), can be used to predict the existence and length of such signal peptides.

Some chaplin proteins may contain a C-terminal cell wall anchoring domain or a transmembrane domain. Preferably the present invention includes chaplin protein variants without such domains. Bioinformatic tools, such as TMHMM by the Center for Biological Sequence Analysis at the Technical University of Denmark, can be used to predict the existence and length of such domains.

The invention also includes variants derived by adding an extra amino acid sequence to a wild-type protein, such as for example, an N-terminal tag, a C-terminal tag or an insertion in the middle of the protein sequence. Non-limiting examples of tags are maltose binding protein (MBP) tag, glutathione S-transferase (GST) tag, thioredoxin (Trx) tag, His-tag, and any other tags known by those skilled in art. Tags can be used to improve solubility and expression levels during fermentation or as a handle for enzyme purification. For example, His6-MBP-TEV_ChpF (SEQ ID NO: 23) is a variant of ChpF (SEQ ID NO: 18) including N-terminal His and MBP tags.

It is important that variants of chaplin proteins retain or preferably improve the ability of the wild-type proteins to adsorb at an interface and to stabilize that interface. Some performance drop in a given property of chaplin protein variants may of course be tolerated, but the chaplin protein variants should retain or preferably improve suitable properties for the relevant application for which they are intended. For instance, screening of variants of one of the wild-types can be used to identify whether they retain or improve appropriate properties.

Suitable examples of chaplin protein variants include one conservative substitution in the peptide, such as a conservative substitution in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 23.

Other suitable examples of chaplin protein variants include 10 or fewer conservative substitutions are included in the peptide, such as five or fewer. The chaplin proteins of the present invention may therefore include 1, 2, 3, 4, 5, 6, 7, 8, 9, 10 or more conservative substitutions. The chaplin proteins can be produced to contain one or more conservative substitutions by manipulating the nucleotide sequence that encodes that peptide using, for example, standard procedures such as site-directed mutagenesis or PCR. Alternatively, the chaplin proteins can be produced to contain one or more conservative substitutions by using peptide synthesis methods, for example, as known in the art.

Examples of amino acids which may be substituted for an original amino acid in a chaplin protein and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

Preferably the chaplin proteins of the invention may comprise variants of SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 17, SEQ ID NO: 18, SEQ ID NO: 19, SEQ ID NO: 20, SEQ ID NO: 21, or SEQ ID NO: 23 wherein one or more cysteine residues are substituted by another amino acid.

Preferably the chaplin proteins of the present invention may comprise variants of SEQ ID NO: 6 or SEQ ID NO: 17, wherein a short amino acid sequence containing two cysteine residues is added at the C-terminus or at least two residues are modified to cysteines. These cysteine residues can allow the chaplin proteins to form multimers (*i.e.*, dimers, tetramers, hexamers and potentially higher order oligomers) in solution due to the formation of disulfide bonds between the cysteine residues of adjacent chaplin protein variants.

The chaplin proteins of the present invention may also cover fragments of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23. Preferably the chaplin protein fragments can adsorb to an interface and stabilize that interface.

The chaplin proteins can be modified by a variety of chemical techniques to produce derivatives having essentially the same or even improved activity as the unmodified peptides, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to

an amide, for example of formula $CONR_1R_2$ wherein $R_1$ and $R_2$ are each independently H or C1-C6 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C6 alkyl or dialkyl amino or further converted to an amide. Hydroxyl groups of the peptide side chains may be converted to alkoxy or ester groups, for example C1-C6 alkoxy or C1-C6 alkyl ester, using well-recognized techniques. Phenyl and phenolic rings of the peptide side chains may be substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the chaplin proteins of the present invention to select and provide conformational constraints to the structure that result in enhanced stability.

Identity, or homology, percentages as mentioned herein in respect of the present invention are those that can be calculated with the GAP program, obtainable from GCG (Genetics Computer Group Inc., Madison, WI, USA). Alternatively, a manual alignment can be performed.

For polypeptide sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps.

A given sequence is typically compared against the full-length sequence of SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23 to obtain a score.

The cleaning composition preferably comprises from 0.001 wt% to 5 wt%, preferably from 0.1 wt% to 1 wt%, by weight of said composition, based on active protein, of one or more chaplin proteins. Preferably said chaplin protein has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23. More preferably said chaplin protein has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one wild-type protein sequence selected from the group consisting of: *Streptomyces coelicolor* ChpD (SEQ ID NO: 16), *Streptomyces coelicolor* ChpE (SEQ ID NO: 17), *Streptomyces coelicolor* ChpF (SEQ ID NO: 18), *Streptomyces coelicolor* ChpG (SEQ ID NO: 19), and *Streptomyces coelicolor* ChpH (SEQ ID NO: 20). Most preferably said chaplin protein has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one wild-type protein sequence selected from the group consisting of: *Streptomyces coelicolor* ChpE (SEQ ID NO: 17) and *Streptomyces coelicolor* ChpF (SEQ ID NO: 18).

Surfactant System

The detergent composition of the invention comprises from 1% to 60%, preferably from 5% to 50%, more preferably from 8% to 40%, by weight of the total composition of a specific surfactant system.

The surfactant system of the composition of the present invention comprises an anionic surfactant. Preferably, the surfactant system for the cleaning composition of the present invention comprises from 1% to 40%, preferably 6% to 35%, more preferably 8% to 30% by weight of the total composition of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sufate and mixtures thereof, and preferably wherein the alkyl alkoxy sulfate is an alkyl ethoxy sulfate. Preferred anionic surfactant is a combination of alkyl sulfates and alkyl ethoxy sulfates with a combined average ethoxylation degree of less than 5, preferably less than 3, more preferably less than 2 and more than 0.5 and an average level of branching of from 5% to 40%, more preferably from 10% to 35%, and even more preferably from 20% to 30%.

The average alkoxylation degree is the mol average alkoxylation degree of all the components of the mixture

(*i.e.*, mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching (\%)} = [(x1 * \text{wt\% branched alcohol 1 in alcohol } 1 + x2 * \text{wt\% branched alcohol 2 in alcohol } 2 + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

The surfactant system of the composition of the present invention further comprises a primary co-surfactant system, wherein the primary co-surfactant system is selected from the group consisting of amphoteric surfactant, zwitterionic surfactant and mixtures thereof. Preferably, the surfactant system for the cleaning composition of the present invention comprises from 0.5% to 15%, preferably from 1% to 12%, more preferably from 2% to 10%, by weight of the total composition of a primary co-surfactant system.

Preferably the primary co-surfactant system is an amphoteric surfactant. Preferably, the primary co-surfactant system is an amine oxide surfactant, and wherein the composition comprises anionic surfactant and amine oxide surfactant in a ratio of less than 9:1, more preferably from 5:1 to 1:1, more preferably from 4:1 to 2:1, preferably from 3:1 to 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or branched alkyl moiety.

Preferably the amine oxide surfactant is a mixture of amine oxides comprising a low-cut amine oxide and a mid-cut amine oxide. The amine oxide of the composition of the invention then comprises:

a) from a10% to 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable exampes of zwitterionic surfactants include betaines, such as alkyl betaines, alkylamidobetaine, amidazoliniumbetaine, sulfobetaine (INCI Sultaines)

as well as the Phosphobetaine and preferably meets formula (I):

R1-[CO-X(CH2)n]x-N+(R2)(R3)-(CH2)m-[CH(OH)-CH2]y-Y- (I)

wherein

R1 is a saturated or unsaturated C6-22 alkyl residue, preferably C8-18 alkyl residue, in particular a saturated C10-16 alkyl residue, for example a saturated C12-14 alkyl residue;
X is NH, NR4 with C1-4 Alkyl residue R4, O or S;
n is a number from 1 to 10, preferably 2 to 5, in particular 3;
x is 0 or 1, preferably 1;
R2 and R3 are independently a C1-4 alkyl residue, potentially hydroxy substituted such as a hydroxyethyl, preferably a methyl;
m is a number from 1 to 4, in particular 1, 2 or 3;
y is 0 or 1; and
Y is COO, SO3, OPO(OR5)O or P(O)(OR5)O, whereby R5 is a hydrogen atom H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 has the same meaning as in formula (I). Particularly preferred betaines are the Carbobetaine [wherein Y-=COO-], in particular the Carbobetaine of the formula (Ia) and (Ib), more preferred are the Alkylamidobetaine of the formula (Ib). A preferred betaine is, for example, Cocoamidopropylbetaine.

Preferably the surfactant system of the composition of the present invention further comprises from 0.1% to 10% by weight of the total composition of a secondary co-surfactant system preferably comprising a non-ionic surfactant. Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (e.g., Simusol® surfactants from Seppic Corporation; and Glucopon® 600 CSUP, Glucopon® 650 EC, Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1.

Enzymes

Preferred compositions of the invention may comprise one or more enzymes selected from the group consisting of amylases, lipases, proteases, cellulases, lipoxygenases, diol synthases, and mixtures thereof. When present in a composition, the aforementioned enzymes may be present at levels from 0.00001 wt% to 2 wt%, from 0.0001 wt% to 1 wt% or from 0.001 wt% to 0.5 wt% by weight of the composition, based on active protein.

Enzyme Stabilizer

Preferably the composition of the invention comprises an enzyme stabilizer. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. Preferably the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to 2 wt% or even up to 1 wt% by weight of the total composition. These salts are formulated from 0.1 to 5 wt%, preferably from 0.2 to 4 wt%, more preferably from 0.3 to 3 wt%, most preferably from 0.5 to 2 wt% relative to the total weight of the composition. Further enzyme stabilizers can be selected from the group (b) carbohydrates selected from the group consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and (g) mixtures thereof.

Salt

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 3 wt%, preferably from 0.05% to 2%, more preferably from 0.2% to 1.5%, or most preferably 0.5% to 1% by weight of said composition, preferably said multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Preferably the cleaning composition further comprises one or more carbohydrates selected from the group comprising derivatives of glucose, mannose, lactose, galactose, allose, altrose, gulose, idose, talose, fucose, fructose, sorbose, tagatose, psicose, arabinose, ribose, xylose, lyxose, ribulose, and xylulose. More preferably the cleaning composition comprises one or more carbohydrates selected from the group of $\alpha$-glucans and $\beta$-glucans. Glucans are polysaccharides of D-glucose monomers, linked by glycosidic bonds. Non-limiting examples of $\alpha$-glucans are dextran, starch, floridean starch, glycogen, pullulan, and their derivatives. Non-limiting examples of $\beta$-glucans are cellulose, chrysolaminarin, curdlan, laminarin, lentinan, lichenin, oat beta-glucan, pleuran, zymosan, and their derivatives.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include

anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See e.g., The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

## Organic Solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more preferably an ethanol, a polyalkyleneglycol, more preferably polypropyleneglycol, and mixtures thereof.

## Amphiphilic Polymer

The composition of the present invention may further comprise from 0.01% to 5%, preferably from 0.05% to 2%, more preferably from 0.07% to 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from 100 to 5,000, preferably from 400 to 2,000, more preferably from 400 to 1,000 Daltons and the alkoxylated polyethyleneimine polymer further comprising:

(i) one or two alkoxylation modifications per nitrogen atom by a polyalkoxylene chain having an average of 1 to 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of 1 to 50 alkoxy moieties per modification wherein the terminal alkoxy moiety is capped with hydrogen, a C1-C4 alkyl or mixtures thereof; or
(iii) a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

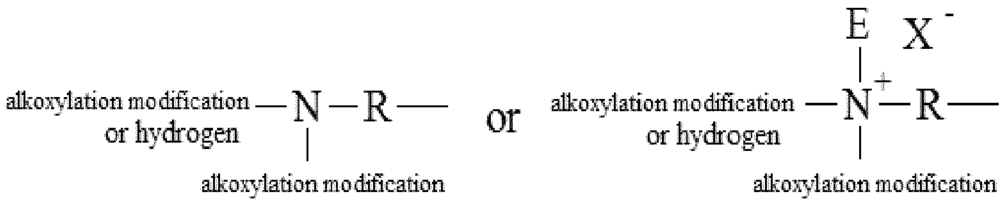

Also, for example, but not limited to, below is shown possible modifications to internal nitrogenatoms in the

polyethyleneimine backbone where R represents an ethylene spacer and E represents a $C_1$-$C_4$ alkyl moiety and X- represents a suitable water soluble counterion.

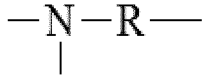
alkoxylation modification

or

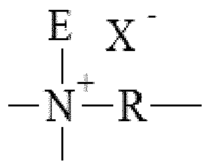
alkoxylation modification

The alkoxylation modification of the polyethyleneimine backbone consists of the replacement of a hydrogen atom by a polyalkoxylene chain having an average of 1 to 50 alkoxy moieties, preferably from 20 to 45 alkoxy moieties, most preferably from 30 to 45 alkoxy moieties. The alkoxy moieties are selected from ethoxy (EO), propoxy (PO),butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from 3 to 30 and an average degree of propoxylation from 1 to 20, more preferably ethoxy/propoxy block moieties having an average degree of ethoxylation from 20 to 30 and an average degree of propoxylation from 10 to 20.

More preferably the ethoxy/propoxy block moieties have a relative ethoxy to propoxy unit ratio between 3 to 1 and 1 to 1, preferably between 2 to 1 and 1 to 1. Most preferably the polyalkoxylene chain is the ethoxy/propoxy block moieties wherein the propoxy moiety block is the terminal alkoxy moiety block.

The modification may result in permanent quaternization of the polyethyleneimine backbone nitrogen atoms. The degree of permanent quaternization maybe from 0% to 30% of the polyethyleneimine backbone nitrogen atoms. It is preferred to have less than 30% of the polyethyleneimine backbone nitrogen atoms permanently quaternized. Most preferably the degree of quaternization is 0%.

A preferred polyethyleneimine has the general structure of Formula (II):

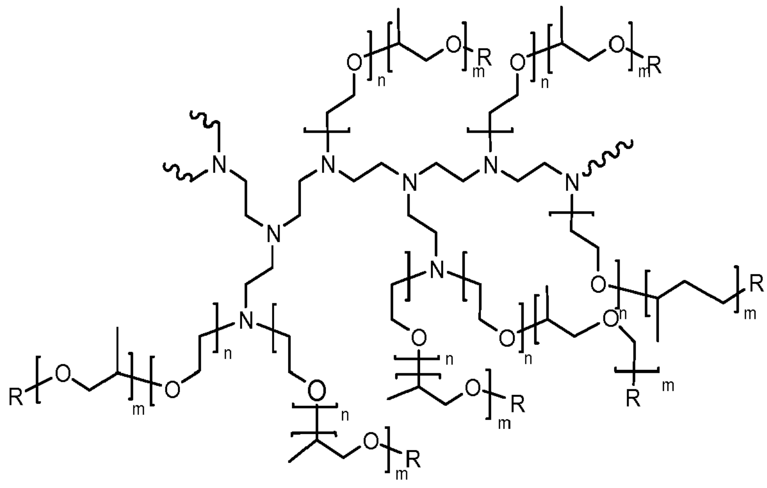
(II)

wherein the polyethyleneimine backbone has a weight average molecular weight of 600, n of formula (II) has an average of 10, m of formula (II) has an average of 7 and R of formula (II) is selected from hydrogen, a $C_1$-$C_4$ alkyl and mixtures thereof, preferably hydrogen. The degree of permanent quaternization of formula (II) may be from 0% to 22% of the polyethyleneimine backbone nitrogen atoms. The molecular weight of this polyethyleneimine preferably is between 10,000 and 15,000.

An alternative polyethyleneimine has the general structure of Formula (II) but wherein the polyethyleneimine backbone has a weight average molecular weight of 600, n of Formula (II) has an average of 24, m of Formula (II) has an average of 16 and R of Formula (II) is selected from hydrogen, a $C_1$-$C_4$ alkyl and mixtures thereof, preferably hydrogen. The degree of permanent quaternization of Formula (II) may be from 0% to 22% of the polyethyleneimine backbone nitrogen atoms. The molecular weight of this polyethyleneimine preferably is between 25,000 and 30,000.

Most preferred polyethyleneimine has the general structure of Formula (II) wherein the polyethyleneimine backbone has a weight average molecular weight of 600, n of Formula (II) has an average of 24, m of Formula (II) has an average of 16 and R of Formula (II) is hydrogen. The degree of permanent quaternization of Formula (II) is 0% of

the polyethyleneimine backbone nitrogen atoms. The molecular weight of this polyethyleneimine preferably is from 25,000 to 30,000, most preferably 28,000.
These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

Chelant

The detergent composition herein can comprise a chelant at a level of from 0.1% to 20%, preferably from 0.2% to 5%, more preferably from 0.2% to 3% by weight of total composition.
As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.
Preferably, the composition of the present invention comprises one or more chelant, preferably selected from the group comprising carboxylate chelants, amino carboxylate chelants, amino phosphonate chelants such as MGDA (methylglycine-N,N-diacetic acid), GLDA (glutamic-N,N- diacetic acid), and mixtures thereof.
Suitable chelating agents can be selected from the group consisting of amino carboxylates, amino phosphonates, polycarboxylate chelating agents and mixtures thereof.
Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Adjunct Ingredients

The cleaning composition herein may optionally comprise a number of other adjunct ingredients such as builders (e.g., preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.*, salt such as NaCl, and other mono-, di- and trivalent salts) and pH adjusters and buffering means (e.g., carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

In another aspect of the invention is directed to a method of washing dishware with the composition of the present invention. The method comprises contacting a cleaning composition with a surface; wherein said cleaning composition comprises a surfactant system and one or more chaplin proteins according to the present invention. As such, the composition herein will be applied in its diluted form to the dishware. Soiled surfaces e.g. dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period

of time ranged from 1 to 10 seconds, although the actual time will vary with each application and user. The contacting of cloth, sponge, or similar article to the surface is preferably accompanied by a concurrent scrubbing of the surface.

In another aspect, the invention is directed to a method of manually washing soiled articles comprising contacting a cleaning composition with a surface, wherein the composition comprises a specific surfactant system and one or more chaplin proteins according to the present invention, and wherein the composition modifies the hydrophobicity of the surface as a result of the contacting step.

Another aspect of the present invention is directed to a method of improving suds longevity or grease emulsification in a washing process for washing soiled articles, preferably dishware or fabric. The method comprises the steps of: a) delivering a cleaning composition comprising a specific surfactant system and one or more chaplin proteins according to the present invention and a surfactant system to a volume of water to form a wash liquor; and b) immersing the soiled articles into said wash liquor. Preferably the chaplin proteins are present at a concentration of 0.005 ppm to 60 ppm, preferably at a concentration of 0.02 ppm to 12 ppm, in an aqueous wash liquor during the washing process

In another aspect, the invention is directed use of one or more chaplin proteins to provide increased suds longevity and/or increase grease emulsification in an aqueous wash liquor during a washing process. Preferably, the aqueous wash liquor further comprises a surfactant system comprising one or more anionic surfactants and one or more co-surfactants selected from the group consisting of amphoteric surfactant, zwitterionic surfactant, and mixtures thereof.

TEST METHODS

The following assays set forth must be used in order that the invention described and claimed herein may be more fully understood.

Test Method 1 - Glass Vial Suds Mileage Method

The objective of the glass vial suds mileage test method is to measure the evolution of suds volume over time generated by a certain solution of detergent composition in the presence of a greasy soil, *e.g.*, olive oil. The steps of the method are as follows:

1. Test solutions are prepared by subsequently adding aliquots at room temperature of: a) 10 g of an aqueous detergent solution at specified detergent concentration and water hardness, b) 1.0 g of an aqueous protein solution at specified concentration and water hardness, and c) 0.11 g of olive oil (Bertolli®, Extra Virgin Olive Oil), into a 40 mL glass vial (dimensions: 95 mm H x 27.5 mm D). For the reference samples, the protein solutions are substituted with 1.0 mL of demineralized water. For the nil detergent samples, the 10g of aqueous detergent solution is replaced by 10 g of water at specified water hardness.
2. The test solutions are mixed in the closed test vials by stirring at room temperature for 2 minutes on a magnetic stirring plate (IKA, model # RTC B S001; VWR magnetic stirrer, catalog # 58949-012; 500 RPM), followed by manually shaking for 20 seconds with an upwards downwards movement (about 2 up and down cycles per second, +/- 30 cm up and 30 cm down).
3. Following the shaking, the test solutions in the closed vials are further stirred on a magnetic stirring plate (IKA, model # RTC B S001; VWR magnetic stirrer, catalog # 58949-012; 500 RPM) for 60 minutes inside a water bath at 46 °C to maintain a constant temperature. The samples are then shaken manually for another 20 seconds as described above and the initial suds heights (H1) are recorded with a ruler.
4. The samples are incubated for an additional 30 minutes inside the water bath at 46 °C while stirring (IKA, model # RTC B S001; VWR magnetic stirrer, catalog # 58949-012; 500 RPM), followed by manual shaking for another 20 seconds as described above. The final suds heights (H2) are recorded.
5. Protein solutions that produce larger suds heights (HI and H2), preferably combined with lower drops in suds height between H1 and H2, are more desirable.

Test Method 2 - Sink Suds Mileage Method

The evolution of the suds volume generated by a solution of a detergent composition can be determined while adding soil loads periodically as follows. A stream of hard water (15 dH) fills a sink (cylinder dimensions: 300 mm D x 288 mm H) to 4 L with a constant pressure of 4 bar. Simultaneously, an aliquot of the detergent composition (final concentration 0.12 w%) is dispensed through a pipette with a flow rate of 0.67 mL/sec at a height of 37 cm above the bottom of the sink surface. An initial suds volume is generated in the sink due to the pressure of the water. The temperature of the solution is maintained at 46 °C during the test.

After recording the initial suds volume (average suds height x sink surface area), a fixed amount of greasy soil (Composition : see Table 1, 6 mL) is injected in the middle of the sink, while a paddle (dimensions: 10 cm x 5 cm,

positioned in the middle of the sink at the air liquid interface at an angle of 45 degrees) rotates 20 times into the solution at 85 RPM. This step is followed immediately by another measurement of the total suds volume. The soil injecting, paddling, and measuring steps are repeated until the measured suds volume reaches a minimum level, which is set at 400 $cm^3$. The amount of soil additions needed to get to that level is recorded. The complete process is repeated a number of times and the average of the number of additions for all the replicates is calculated for each detergent composition

Finally, the suds mileage index is then calculated as: (average number of soil additions for test detergent composition) / (average number of soil additions for reference detergent composition) x 100.

Pending on the test purpose the skilled person could choose to select an alternative water hardness, solution temperature, product concentration or soil type.

Table 1 - Greasy Soil Composition

| Ingredient | Weight % |
|---|---|
| Crisco oil | 12.730 |
| Crisco shortening | 27.752 |
| Lard | 7.638 |
| Refined Rendered Edible Beef Tallow | 51.684 |
| Oleic Acid, 90% (Techn) | 0.139 |
| Palmitic Acid, 99+% | 0.036 |
| Stearic Acid, 99+% | 0.021 |

EXAMPLES

The following examples are provided to further illustrate the present invention and are not to be construed as limitations of the present invention, as many variations of the present invention are possible without departing from its spirit or scope.

Example 1a - Production of *Streptomyces coelicolor* A3(2) ChpE

Chaplin ChpE without the N-terminal signal peptide (SEQ ID NO: 17) is chemically synthesized (Genscript; Piscataway, NJ) by solid phase peptide synthesis using standard protocols known in the art to obtain a material with 92.9 w% purity as determined by HPLC analysis.

Example 1b - Production of *Streptomyces coelicolor* A3(2) ChpF

A codon optimized gene (SEQ ID NO: 22) encoding for *Streptomyces coelicolor* A3(2) ChpF (SEQ ID NO: 18) is designed and synthesized. After synthesis, the gene is subcloned into a modified pET28a vector for heterologous expression of a ChpF variant including an additional N-terminal region containing a His-tag, a MBP tag, and a TEV protease cleavage site (SEQ ID NO: 23). The protein is expressed and purified by Genscript (Piscataway, NJ). In brief, *Escherichia coli* BL21 (DE3) cells are transformed with the recombinant plasmid and a single colony is inoculated into TB medium containing the proper kanamycin. Cultures are incubated at 15 °C for 16 h at 200 rpm and isopropyl β-D-1-thiogalactopyranoside (IPTG) was added (final concentration 1 mM) to induce protein expression. Cells are harvested by centrifugation and the pellets are lysed by sonication. After centrifugation, the supernatant is collected and the protein is purified by one-step purification using a nickel affinity column and standard protocols known in the art. The protein is stored in a buffer containing 50 mM Tris-HCl, 150 mM NaCl, and 10% Glycerol at pH 8.0. The final protein concentration is 1.30 mg/ mL as determined by Bradford protein assay with BSA as a standard (ThermoFisher, catalog # 23236).

Example 1c - Detergent Compositions

The evolution of suds volume generated by a certain solution of detergent composition in presence of a soil, *i.e.*, olive oil or greasy soil, is followed over time under specific conditions (*e.g.*, water hardness, solution temperature, detergent concentrations, etc.). The following solutions are prepared:

A. Hard water (15 dH): 0.75 g $MgCl_2.6H_2O$ (Sigma-Aldrich, catalog # M9272), 2.10 g $CaCl_2.6H_2O$ (Sigma-Aldrich, catalog # 21108), and 0.689 g $NaHCO_3$ (Sigma-Aldrich, catalog # 31437) are dissolved in 5 L of demineralized water.
B. Detergent solution of a high surfactant content detergent composition ("solution DG-HS") is prepared using Fairy Dark Green, as commercially available in the UK in Feb 2017, diluted in hard water (15 dH) prepared as above, at targeted detergent concentration of 0.12%.
C. Detergent solution of a low surfactant content detergent composition ("solution DG-LS") is prepared using Fairy Dark Green, as commercially available in the UK in Feb 2017, diluted in hard water (15 dH) prepared as above, at targeted detergent concentration of 0.06%.
D. Protein solutions: Proteins are diluted in demineralized water to the required concentration before proceeding with the suds mileage method.
E. Greasy soil: A grease soil is prepared according to the composition described in Table 1.

Example 2 - Glass Vial Suds Mileage of *Streptomyces coelicolor* A3(2) ChpE with Olive Oil

Inventive Compositions A, B and C are examples of cleaning compositions according to the present invention, made with: a) detergent solution DG-LS (prepared as described in Example 1c), and b) diluted samples of purified *Streptomyces coelicolor* A3 (2) ChpE (prepared as described in Example 1a). Comparative Composition D contains the same detergent solution DG-LS in the absence of the chaplin protein. Comparative Composition E contains a diluted sample of purified *Streptomyces coelicolor* A3(2) ChpE in the absence of the detergent solution DG-LS (replaced with hard water - 15dH). The glass vial suds mileage test is performed on the compositions using olive oil as described in the test methods section (Test Method 1). The initial (H1) and final (H2) measurements are recorded in Table 2. The % suds height drop represents the drop in suds height as measured between the initial and final time point and is calculated by the following equation:

$$\% \text{ suds height drop} = \{(H1 - H2)/H1\} * 100.$$

The % suds height drops are calculated for the compositions and shown in Table 2.

Table 2: Suds Mileage

| Compositions | ChpE Concentration in Composition [ppm] | H1 [mm] | H2 [mm] | % suds height drop H2 vs H1 |
|---|---|---|---|---|
| Inventive Composition A | 60 | 7 | 7 | 0% |
| Inventive Composition B | 24 | 6 | 5 | 17% |
| Inventive Composition C | 12 | 5 | 4 | 20% |
| Comparative Composition D | 0 | 4 | 3 | 25% |
| Comparative Composition E | 60 | 0 | 0 | not applicable (no suds) |

The results confirm that Inventive Compositions A-C detergent solutions comprising *Streptomyces coelicolor* A3(2) ChpE according to the invention (SEQ ID NO: 17) have a superior suds profile compared to Comparative Composition D solution comprising the specific surfactant system according to the invention but without the chaplin protein according to the invention, both in view of absolute suds height build-up as in view of sustaining the suds height in presence of greasy soil. Comparative Composition E comprising *Streptomyces coelicolor* A3(2) ChpE according to the

invention (SEQ ID NO: 17) without the specific surfactant system according to the invention produced no suds, illustrating a suds a synergistic suds boost between the protein and the specific surfactant system.

Example 3 - Glass Vial Suds Mileage of *Streptomyces coelicolor* A3(2) ChpF with Olive Oil

Inventive Compositions F and G are examples of cleaning compositions according to the present invention, made with: a) detergent solution DG-LS (prepared as described in Example 1c), b) diluted samples of purified *Streptomyces coelicolor* A3(2) ChpF (prepared as described in Example 1b). Comparative Composition H contains the same detergent solution DG-LS in the absence of the chaplin protein. Comparative Composition I contains diluted samples of purified *Streptomyces coelicolor* A3(2) ChpF in the absence of the detergent solution DG-LS (replaced with hard water - 15dH). The glass vial suds mileage test is performed on these compositions using: olive oil as described in the test methods section (Test Method 1). The initial (H1) and final (H2) measurements are recorded in Table 3. The % suds height drops are calculated for the compositions and shown in Table 3.

Table 3: Suds Mileage

| Compositions | ChpF Concentration in Composition [ppm] | H1 [mm] | H2 [mm] | % suds height drop H2 vs H1 |
|---|---|---|---|---|
| Inventive Composition F | 60 | 7 | 7 | 0% |
| Inventive Composition G | 12 | 6 | 5 | 17% |
| Comparative Composition H | 0 | 5 | 3 | 40% |
| Comparative Composition I | 60 | 0 | 0 | not applicable (no suds) |

The results confirm that Inventive Compositions F and G detergent solutions comprising *Streptomyces coelicolor* A3(2) ChpF (*e.g.*, SEQ ID NO: 18) or its variants (*e.g.*, SEQ ID NO: 23) according to the invention have a superior suds profile compared to Comparative Composition H solution comprising the specific surfactant composition according to the invention but without the chaplin protein according to the invention, both in view of absolute suds height build-up as in view of sustaining the suds height in presence of greasy soil. Comparative Composition I comprising *Streptomyces coelicolor* A3(2) ChpF according to the invention (SEQ ID NO: 18) without the specific surfactant system according to the invention produced no suds, illustrating as such the synergistic suds boost between the protein and the specific surfactant system according to the invention.

Example 4 - Exemplary Manual Dish-Washing Detergent Composition

Table 4 exemplifies a manual dish-washing detergent composition comprising *Streptomyces coelicolor* A3(2) ChpE (SEQ ID NO: 17) or *Streptomyces coelicolor* A3(2) ChpF (SEQ ID NO: 18) or His6-MBP-TEV_ChpF (SEQ ID NO: 23) proteins according to the invention.

Table 5: Detergent Composition

| Ingredient | Wt% |
|---|---|
| Sodium alkyl ethoxy sulfate (C1213E00.6S) | 22.91% |
| n-C12-14 Di Methyl Amine Oxide | 7.64% |
| Lutensol XP80 (non-ionic surfactant supplied by BASF) | 0.45% |
| Sodium Chloride | 1.2% |
| Poly Propylene Glycol (MW 2000) | 1% |
| Ethanol | 2% |
| Sodium Hydroxide | 0.24% |

(continued)

| Ingredient | Wt% |
|---|---|
| *Streptomyces coelicolor* A3(2) ChpE (SEQ ID NO: 17) or *Streptomyces coelicolor* A3(2) ChpF (SEQ ID NO: 18) or His6-MBP-TEV_ChpF (SEQ ID NO: 23) | 0.5% |
| Minors (perfume, preservative, dye) + water | To 100 % |
| pH (@ 10% solution) | 9 |

All percentages and ratios given for proteins are based on active protein. All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A cleaning composition comprising:

a) from 1 wt% to 60 wt%, preferably from 5 wt% to 50 wt%, by weight of the cleaning composition of a surfactant system comprising one or more anionic surfactants and one or more co-surfactants selected from the group consisting of amphoteric surfactant, zwitterionic surfactant, and mixtures thereof; and
b) from 0.001 wt% to 5 wt%, preferably from 0.1 wt% to 1 wt%, by weight of the cleaning composition, based on active protein, of one or more chaplin proteins, wherein the chaplin proteins comprise a DUF320 domain consensus sequence (SEQ ID NO: 1) and have a sequence of less than 350 amino acids;

preferably wherein the cleaning composition is a liquid manual dishwashing cleaning composition.

**2.** The cleaning composition according to claim 1, wherein the chaplin proteins have at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23, preferably to at least one wild-type protein sequence selected from the group consisting of: *Streptomyces coelicolor* ChpD (SEQ ID NO: 16), *Streptomyces coelicolor* ChpE (SEQ ID NO: 17), *Streptomyces coelicolor* ChpF (SEQ ID NO: 18), *Streptomyces coelicolor* ChpG (SEQ ID NO: 19), and *Streptomyces coelicolor* ChpH (SEQ ID NO: 20), more preferably *Streptomyces coelicolor* ChpE (SEQ ID NO: 17) and *Streptomyces coelicolor* ChpF (SEQ ID NO: 18).

**3.** The composition according to any preceding claims, further comprising one or more carbohydrates selected from the group comprising O-glycan, N-glycan, and mixtures thereof.

**4.** The composition according to any preceding claims, wherein the weight ratio of the anionic surfactants to the co-surfactants is less than 9:1, more preferably from 5:1 to 1:1, more preferably from 4:1 to 2:1.

**5.** The composition according to any preceding claims, wherein the amphoteric surfactant is amine oxide surfactant and said zwitterionic surfactant is betaine surfactant.

**6.** The composition according to any preceding claims, wherein the anionic surfactants are selected from the group consisting of: alkyl sulfates, alkyl alkoxy sulfates, alkyl benzene sulfonates, paraffin sulfonates, and mixtures thereof, preferably a mixture of alkyl sulfates and alkyl ethoxy sulfates.

**7.** The composition according to any preceding claims, wherein the anionic surfactants are a mixture of alkyl sulfates

and alkyl alkoxy sulfates, wherein the co-surfactants are alkyl dimethyl amine oxides, and wherein the weight ratio of the anionic surfactants to the co-surfactants is from 4:1 to 2:1.

**8.** The composition according to any preceding claims, further comprising a chelant, preferably selected from the group comprising carboxylate chelants, amino carboxylate chelants, amino phosphonate chelants, and mixtures thereof, preferably selected from the group of MGDA (methylglycine-N,N-diacetic acid), GLDA (glutamic-N,N- diacetic acid), and mixtures thereof.

**9.** The composition according to any preceding claims, further comprising one or more enzymes selected from the group consisting of amylases, lipases, proteases, cellulase, lipoxygenases, diol synthases, and mixtures thereof.

**10.** A method comprising contacting a cleaning composition with a surface preferably dishware; wherein the cleaning composition comprises a surfactant system comprising one or more anionic surfactants and one or more co-surfactants selected from the group consisting of amphoteric surfactant, zwitterionic surfactant, and mixtures thereof; and one or more chaplin proteins, wherein the chaplin proteins comprise a DUF320 domain consensus sequence (SEQ ID NO: 1) and have a sequence of less than 350 amino acids, preferably wherein the chaplin proteins have at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23, more preferably to at least one wild-type protein sequence selected from the group consisting of: *Streptomyces coelicolor* ChpD (SEQ ID NO: 16), *Streptomyces coelicolor* ChpE (SEQ ID NO: 17), *Streptomyces coelicolor* ChpF (SEQ ID NO: 18), *Streptomyces coelicolor* ChpG (SEQ ID NO: 19), and *Streptomyces coelicolor* ChpH (SEQ ID NO: 20), more preferably *Streptomyces coelicolor* ChpE (SEQ ID NO: 17) and *Streptomyces coelicolor* ChpF (SEQ ID NO: 18).

**11.** A method of manually washing soiled items preferably dishware comprising contacting a cleaning composition according to claims 1 to 9 with a surface, wherein said chaplin proteins have at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23, more preferably to at least one wild-type protein sequence selected from the group consisting of: *Streptomyces coelicolor* ChpD (SEQ ID NO: 16), *Streptomyces coelicolor* ChpE (SEQ ID NO: *17), Streptomyces coelicolor* ChpF (SEQ ID NO: 18), *Streptomyces coelicolor* ChpG (SEQ ID NO: 19), and *Streptomyces coelicolor* ChpH (SEQ ID NO: 20), more preferably *Streptomyces coelicolor* ChpE (SEQ ID NO: 17) and *Streptomyces coelicolor* ChpF (SEQ ID NO: 18), wherein the composition modifies the hydrophobicity of the surface as a result of the contacting step.

**12.** A method of improving suds longevity or grease emulsification in a washing process for washing soiled articles, preferably dishware, comprising the steps of:

a) delivering a cleaning composition to a volume of water to form a wash liquor; and
b) immersing the soiled articles into said wash liquor;

wherein said cleaning composition comprises one or more chaplin proteins wherein the chaplin proteins comprise a DUF320 domain consensus sequence (SEQ ID NO: 1) and have a sequence of less than 350 amino acids, preferably wherein the chaplin proteins have at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 or SEQ ID NO: 23, more preferably to at least one wild-type protein sequence selected from the group consisting of: *Streptomyces coelicolor* ChpD (SEQ ID NO: 16), *Streptomyces coelicolor* ChpE (SEQ ID NO: 17), *Streptomyces coelicolor* ChpF (SEQ ID NO: 18), *Streptomyces coelicolor* ChpG (SEQ ID NO: 19), and *Streptomyces coelicolor* ChpH (SEQ ID NO: 20), more

preferably *Streptomyces coelicolor* ChpE (SEQ ID NO: 17) and *Streptomyces coelicolor* ChpF (SEQ ID NO: 18).

**13.** The method according to claim 12, wherein the chaplin proteins are present at a concentration from 0.005 ppm to 60 ppm, preferably from 0.02 ppm to 12 ppm, based on active protein, in an aqueous wash liquor during the washing process.

**14.** Use of one or more chaplin proteins to provide improved suds longevity and/or improved grease emulsification in an aqueous wash liquor during a washing process, wherein the chaplin proteins comprise a DUF320 domain consensus sequence (SEQ ID NO: 1) and have a sequence of less than 350 amino acids.

**15.** The use according to claim 14 wherein said chaplin proteins have at least 80%, preferably at least 85%, preferably at least 90%, preferably at least 95%, preferably at least 98% or even 100% amino acid identity as calculated over the entire length of the sequence aligned against the entire length of at least one reference sequence selected from the group consisting of SEQ ID NO: 2, SEQ ID NO: 3, SEQ ID NO: 4, SEQ ID NO: 5, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, or SEQ ID NO: 23, more preferably to at least one wild-type protein sequence selected from the group consisting of: *Streptomyces coelicolor* ChpD (SEQ ID NO: 16), *Streptomyces coelicolor* ChpE (SEQ ID NO: 17), *Streptomyces coelicolor* ChpF (SEQ ID NO: 18), *Streptomyces coelicolor* ChpG (SEQ ID NO: 19), and *Streptomyces coelicolor* ChpH (SEQ ID NO: 20), more preferably *Streptomyces coelicolor* ChpE (SEQ ID NO: 17) and *Streptomyces coelicolor* ChpF (SEQ ID NO: 18).

Europäisches Patentamt
European Patent Office
Office européen des brevets

# EUROPEAN SEARCH REPORT

Application Number
EP 18 17 8727

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/031272 A1 (SHIPOVSKOV STEPAN [DK] ET AL) 30 January 2014 (2014-01-30) * paragraphs [0001], [0003], [0007], [0008], [0011] - [0017], [0145], [0419] - [0421], [0431] - [0433] * ----- | 1-11 | INV. C11D3/38 C11D1/94 C11D11/00 C07K14/36 |
| A | WO 00/71658 A1 (PROCTER & GAMBLE [US]; KASTURI CHANDRIKA [US]; SCHAFER MICHAEL GAYLE [] 30 November 2000 (2000-11-30) * page 1, line 1 - page 3, paragraph 1 * * page 3 * * page 4, paragraph 2 * * page 5, last paragraph - page 7, paragraph 2 * * page 35, last paragraph - page 39, paragraph 2; examples * ----- | 12-15 | |
| A | SCHOR MARIEKE ET AL: "The Diverse Structures and Functions of Surfactant Proteins", TRENDS IN BIOCHEMICAL SCIENCES, ELSEVIER, AMSTERDAM, NL, vol. 41, no. 7, 27 May 2016 (2016-05-27), pages 610-620, XP029624822, ISSN: 0968-0004, DOI: 10.1016/J.TIBS.2016.04.009 * the whole document * ----- | 1-15 | TECHNICAL FIELDS SEARCHED (IPC) C11D C07K |

The present search report has been drawn up for all claims

1

| Place of search | Date of completion of the search | Examiner |
|---|---|---|
| The Hague | 17 October 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 18 17 8727

This annex lists the patent family members relating to the patent documents cited in the above-mentioned European search report. The members are as contained in the European Patent Office EDP file on The European Patent Office is in no way liable for these particulars which are merely given for the purpose of information.

17-10-2018

| Patent document cited in search report | Publication date | Patent family member(s) | Publication date |
|---|---|---|---|
| US 2014031272 A1 | 30-01-2014 | AR 085845 A1 | 30-10-2013 |
| | | AU 2012241055 A1 | 15-08-2013 |
| | | BR 112013025811 A2 | 29-11-2016 |
| | | CA 2830579 A1 | 11-10-2012 |
| | | EP 2694537 A1 | 12-02-2014 |
| | | JP 6027092 B2 | 16-11-2016 |
| | | JP 2014516509 A | 17-07-2014 |
| | | KR 20140024365 A | 28-02-2014 |
| | | RU 2013149861 A | 20-05-2015 |
| | | US 2014031272 A1 | 30-01-2014 |
| | | WO 2012137147 A1 | 11-10-2012 |
| WO 0071658 A1 | 30-11-2000 | AR 020297 A1 | 02-05-2002 |
| | | AU 5444100 A | 12-12-2000 |
| | | BR 0011549 A | 26-02-2002 |
| | | CA 2372894 A1 | 30-11-2000 |
| | | CA 2688927 A1 | 30-11-2000 |
| | | EP 1180132 A1 | 20-02-2002 |
| | | JP 2003500525 A | 07-01-2003 |
| | | MX PA01012189 A | 30-07-2002 |
| | | WO 0071658 A1 | 30-11-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201219844 A
- WO 201219849 A
- WO 201219848 A
- US 3915903 A
- WO 2007135645 A


### Non-patent literature cited in the description


- **COOPER, A. et al.** *Colloids Surf., A: Physiochemical and Engineering Aspects,* 2017
- **SCHOR, M. et al.** *Trends Biochem. Sci.,* 2016, vol. 41 (7), 610-620
- **PETERSEN TN. ; BRUNAK S. ; HEIJNE G. ; NIELSEN H.** *Nature Methods,* 2011, vol. 8, 785-786
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **ROBERT LAUGHLIN.** The Aqueous Phase Behaviour of Surfactants. Academic Press, 1994, 538-542